# EUROPÄISCHE PATENTSCHRIFT

(11) **EP 1 210 945 B1**
(45) Veröffentlichungstag und Bekanntmachung des Hinweises auf die Patenterteilung: **10.05.2006**
(21) Anmeldenummer: 01128629.1
(22) Anmeldetag: 30.11.2001
(51) Int. Cl.: A61P 9/10, A61K 36/185, A61K 36/76, A61K 125/00, A61K 129/00

(54) **Arzneimittelzubereitung mit antiarteriosklerotischer Wirkung**
Pharmaceutical composition with antiarteriosclerotic activity
Composition pharmaceutique anti-artériosclérotique

(30) Priorität: 01.12.2000 DE 10059838
(43) Veröffentlichungstag der Anmeldung: 05.06.2002
(73) Patentinhaber: Salus-Haus GmbH & Co. KG, 83052 Bruckmühl (DE)
(72) Erfinder: Greither, Otto, 83052 Bruckmühl (DE)
(74) Vertreter: Becker Kurig Straus

(56) Entgegenhaltungen:
- FR-A- 2 614 791
- DATABASE BIOSIS [Online] BIOSCIENCES INFORMATION SERVICE, PHILADELPHIA, PA, US; 1984 CIRCOSTA C ET AL: "A DRUG USED IN TRADITIONAL MEDICINE HARPAGOPHYTUM-PROCUMBENS 2. CARDIOVASCULAR ACTIVITY" Database accession no. PREV198579033845 XP002194523 & JOURNAL OF ETHNOPHARMACOLOGY, Bd. 11, Nr. 3, 1984, Seiten 259-274, ISSN: 0378-8741

## Beschreibung

Die Erfindung betrifft den in den Patentansprüchen angegebenen Gegenstand. Die Erfindung betrifft insbesondere eine neue Verwendung von Extrakten aus Teufelskrallenwurzeln (Harpagophytum procumbens), gegebenenfalls in Kombination mit Extrakten aus Salicis cortex (Weidenrinde).

Arteriosklerose, umgangssprachlich auch häufig als Arterienverkalkung bezeichnet, betrifft die wichtigste und häufigste krankhafte Veränderung der Arterien mit Verhärtung, Verdickung, Elastizitätsverlust und Lichtungseinengung der Arterien. Für die Auslösung der Arteriosklerose werden zahlreiche exogene und endogene Noxen bzw. Krankheiten verantwortlich gemacht. Aufgrund von tierexperimentellen und klinischen Beobachtungen hat man z.B. Hypertonie, Hyperlipidämie, Diabetes mellitus, Toxine, Nikotin, antigen-antikörper-komplexe Entzündungen, Hypoxie, Wirbelbindungen, psychischen Stress, Alter und familiäre Belastungen verantwortlich gemacht. Die Pathogenese der Arteriosklerose lässt sich grob in drei Stadien einteilen. Im ersten Stadium tritt eine Veränderung des Gefäßinhaltes auf. Druck und Wirbelhyperlipidämie bewirken Läsionen des Endothels und steigern einen Stoffeinstrom. Dadurch werden metabolische und zelluläre Reaktionen der Zellwand ausgelöst, nämlich Intima-Ödem, Synthesesteigerung saurer Mukopolysaccharide, Ausfällung von Lipoproteinen, Fibrinogen und Albumin. Die Bindegewebs- und Muskelzellen reagieren mit einer Proliferation und gesteigerter Kollagen- und Elastinsynthese, evtl. auch Hyalinose. Begleitend hierzu ist auch eine zunehmende Lipidose, oft Nekrose, Ulceration und Verkalkung festzustellen. Im zweiten Stadium kann man primäre Veränderungen der Intima oder des Endothelfilms mit Ablagerungen von Blutplättchen feststellen. Häufig findet man auch sklerotische Beete als organisierte parietale Thromben. Schließlich wird eine Lipidose der Gefäßwand als sekundäre Veränderung festgestellt.

Im dritten Stadium findet sich die Arteriosklerose als Folge einer primären krankhaften Veränderung der Gefäßwand. Blutdruckerhöhung und Hyperlipidämie können den Prozess intensivieren. Die intimalen Myozyten, bzw. Bindegewebszellen, proliferieren aus noch unbekannter Ursache und bilden vermehrt kollagene Fasern und Muco-Polysaccharide sowie Lipide.

Nach neueren Erkenntnissen wird der Wechselwirkung zwischen Thrombozyten und der Gefäßwand eine maßgebliche Rolle in der Pathogenese der Arteriosklerose beigemessen. Eine Rückbildung der Frühstadien der Arteriosklerose lässt sich beispielsweise durch Ausschaltung bzw. Reduktion von arterogenen Noxen erreichen.

Bislang wurden jedoch noch keine Arzneimittel gefunden, die eine Rückbildung der Frühstadien der Arteriosklerose bewirken, bzw. mittlere Stadien zurückbilden helfen. Bei bekannten prophylaktisch anzuwendenden Naturheilmitteln, wie z.B. Knoblauch-Extrakt ist jedoch die Wirkung noch nicht eindeutig nachgewiesen worden.

Schon sehr lange werden Teezubereitungen, bzw. Extrakte aus Teufelskrallewurzeln (Radix harpagophytii) bei dyspeptischen Beschwerden bzw. zur Behandlung rheumatischer Erkrankungen eingesetzt. In FR-A-2614791 wird eine Zusammensetzung aus 400 ml Harpagophytum procumbens, 20 mg Selen und 25 mg Zink zur Behandlung von Rheumatismus und Entzündungen beschrieben.

In dem Artikel von Circosta C et al. im Journal of Ethnopharmacology, Bd. 11, Nr. 3, 1984, Seiten 259 bis 274, zitiert als Zusammenfassung, wird von Effekten von Extrakten aus Harpagophytum procumbens bei normotensiven Ratten berichtet. Der eingesetzte H. procumbens Extrakt zeigte eine protektive Wirkung im Bezug durch Aconitin, und insbesondere durch Calciumchlorid und Epinephrin-Chloroform induzierte Arrhythmien. Ein methanolischer Extrakt vom H. procumbens verursachte nämlich eine signifikante, dosisabhängige Reduktion des arteriellen Blutdrucks bei normotensiven Ratten.

Den Erfahrungsberichten und klinischen Beobachtungen trug die Kommission E des ehemaligen Bundesgesundheitsamtes Rechnung, indem sie 1989 eine positive Monographie "Radix harpagophytii" veröffentlichte. Danach werden Zubereitungen aus Teufelskrallewurzeln bei dyspeptischen Beschwerden eingesetzt (Tagesdosis 1,5 g Droge) und zur unterstützenden Behandlung degenerativer Erkrankungen des Bewegungsapparats (Tagesdosis 4,5 g Droge) (Bundesanzeiger Nr. 43 vom 2. März 1989). Die Wirksamkeit konnte bisher noch nicht bestimmten Inhaltsstoffen der Teufelskrallenwurzel zugeordnet werden.

Die südafrikanische Teufelskralle Harpagophytum procumbens zählt zur Familie der Pedaliaceae (Sesamgewächse) und ist in den Steppengebieten Süd- und Südwestafrikas heimisch. Die Bezeichnung "Teufelskralle" rührt von den verholzten und dornigen Früchten der Pflanze her, die Verzweigungen bilden und mit Widerhaken versehen sind, woraus dann die "Kralle des Teufels" wurde. Der arzneilich interessante Teil der Pflanze ist allerdings die Wurzelknolle. In diesen großen, bis zu 600 g schweren und ca. 50 cm langen Speicherwurzeln von Harpagophytum procumbens sind die Inhaltsstoffe enthalten. Als wesentliche Inhaltsstoffe werden Iridoidglycoside, insbesondere Harpagosid, daneben Harpagid und Procumbid angesehen. Weiterhin sind große Mengen Zucker, Fette, Wachse und Stearine enthalten. Als Leitsubstanz wird das Harpagosid angesehen.

Im Handel befinden sich insbesondere Teezubereitungen aus Teufelskrallenwurzel-Extrakten sowie oral einzunehmende Kapsel- und Tablettenpräparate, die einfache wässerige und wässerigalkoholische Extrakte aus Teufelskrallewurzeln enthalten. Diese Präparate werden als Adjuvans zur Therapie von schmerzhaften degenerativen Beschwerden des Bewegungsapparates angeboten und verwendet.

Weiterhin konnte in klinischen Doppelblindstudien eine antirheumatische Wirksamkeit von Teufelskrallenwurzel-Extrakten gezeigt werden. Dabei wurden Extrakte eingesetzt, die eine Dosierung von 50 mg Harpagosid pro Tag sicherstellten. Die üblichen pharmazeutischen Präparate mit einfachen Extrakten aus Teufelskrallewurzeln weisen Harpagosidgehalte von 1,2 bis 3 Gew.-% auf. Zur Gewährleistung einer Zuführung von 50 mg Harpagosid pro Tag sind daher Extraktmengen von 1500-4500 mg erforderlich.

DE 19603788 A1 beschreibt ein Verfahren zur Herstellung eines hochkonzentrierten Extrakts von Wirkstoffen aus der Teufelskralle, wobei ein roher, wässeriger Extrakt mit einem nicht ionogenen Adsorberharz in Berührung gebracht wird und die daran gebundenen Wirkstoffe in einer zweiten Stufe durch Behandlung mit einem Alkohol oder einem ähnlichen physiologisch unbedenklich Lösungsmittel gelöst und der so gewonnene Extrakt unter schonenden Bedingungen konzentriert wird. Die Extrakte eignen sich zur Herstellung eines Monotherapeutikums, insbesondere zur Behandlung von schmerzhaften Beschwerden des menschlichen oder tierischen Bewegungsapparates.

DE 19 651 290 beschreibt ein Verfahren zur Herstellung von Extrakten aus Teufelskrallewurzeln mit einem hohen Gehalt an Harpagosid. Die beschriebenen Extrakte können unter Zusatz üblicher pharmazeutischer Hilfsstoffe in bekannter Weise zu oral applizierbaren festen oder flüssigen Arzneizubereitungen mit antirheumatischer und antiphlogistischer Wirkung verarbeitet werden.

Von Weidenrindenpulver ist bekannt, dass es sehr gute Wirkungen gegen Fieber, fieberhafte Erkrankungen, rheumatische Beschwerden und Kopfschmerzen zeigt. In der Volksheilkunde wird allgemein bei grippalen Zuständen, Zahnschmerzen, zur Behandlung leichter Schmerzen, äußerlich bei Fußschweiß und zur Behandlung schlecht heilender Wunden Weidenrindenpulver bzw. Tees aus Weidenrindenpulver verabreicht. Bei der Droge aus Salix cortex (Weidenrinde) handelt es sich um im Frühjahr gesammelte, ganze, geschnittene oder pulverisierte, getrocknete Rinde junger Zweige mit einem Mindestgesamt-Salicin-Gehalt von 1,0 Gew.-%. Die Droge stammt überwiegend aus dem ehemaligen Jugoslawien, Bulgarien, Ungarn und Rumänien. Als Inhaltsstoffe sind Phenolglycoside, überwiegend Ester des Salicins, bekannt, darunter als Hauptverbindungen Salicortin, Tremolacin und 2'-Acetylsalicortin. Die Mengenverhältnisse und der absolute Gehalt innerhalb der Gattung schwanken stark und betragen je nach Art zwischen 1,5 und 11 Gew.-%. Als mittlere Tagesdosis für flüssige und feste Darreichungsformen zur innerlichen Anwendung für die oben angesprochenen Fälle wird eine Gesamtsalicinmenge von 60-120 mg angesehen. Zur Teebereitung existieren zahlreiche Vorschriften. Bevorzugt wird ein Kaltwasserauszug. Dazu werden 1-2 Teelöffel Weidenrinde mit 1-2 Tassen Wasser angesetzt, über Nacht ziehen gelassen und dann tags darauf getrunken.

DE 3 832 277 A1 beschreibt Brausetabletten, die als pharmazeutischen Wirkstoff einen Pflanzenextrakt aus Weidenrinde enthalten. Gegenüber bekannten Schmerzmitteltabletten auf Basis von Acetylsalicylsäure (ASS) bieten weidenrindenextrakthaltige Brausetabletten den Vorteil, dass die ASS-typischen Nebenwirkungen an der Magenschleimhaut beim Weidenrinde-Extrakt nicht auftreten. Der strenge Geschmack von ASS ist außerdem weniger vorhanden und es handelt sich beim Weidenrinde-Extrakt um ein Naturprodukt, das gegenüber synthetisch hergestellten Pharmazeutika im Markt eine zunehmende Akzeptanz erfährt.

Der vorliegenden Erfindung liegt daher die Aufgabe zugrunde, eine neue Arzneimittelzubereitung mit anti-arteriosklerotischer Wirkung auf Basis natürlicher Wirkstoffe bereitzustellen.

Diese Aufgabe wird erfindungsgemäß durch eine Arzneimittelzubereitung mit antiarteriosklerotischer Wirkung gelöst, enthaltend als Wirkstoff einen Extrakt aus Harpagophytum procumbens (Teufelskralle), gegebenenfalls in Kombination mit Extrakten aus Salicis cortex (Weidenrinde).

In den Unteransprüchen sind vorteilhafte, jedoch nicht einschränkende, Ausführungsformen enthalten.

Erfindungsgemäß eigenen sich die Extrakte von Harpagophytum procumbens (Teufelskralle), gegebenenfalls in Kombination mit Weidenrinde-Extrakten zur Herstellung eines Therapeutikums, insbesondere zur Prophylaxe und Behandlung von Arteriosklerose. Diese werden bevorzugt oral, in fester oder flüssiger Form, verabreicht.

In einer erfindungsgemäßen Ausführungsform ist es aber auch möglich, in die Arzneimittelzubereitung als weiteren Wirkstoff Trockenextrakte aus Salicis cortex (Weidenrinde) einzubringen. Die Drogen der Weidenrinde, d.h. überwiegend die Ester des Salicins, darunter die Hauptverbindungen Salicortin, Tremolacin und 2'-Acetylsalicortin bewirken zusätzlich zu dem Teufelskralle-Extrakt, dass die Heilung bei Arteriosklerose im Frühstadium verstärkt, bzw. die Krankheit rückgebildet wird (synergistischer Effekt).

Die vorliegende Erfindung beruht auf dem überraschenden Befund, dass Trockenextrakte aus Harpagophytum procumbens die Neointima-Bildung, welche auf eine endothele, denudierende Verletzung einer Oberschenkelarterie folgt, vorteilhafterweise moduliert.

Es wurde nämlich festgestellt, dass die fortwährende Verabreichung von den erfindungsgemäßen Trockenextrakten aus Harpagophytum procumbens (Teufelskralle) in einer großen Verbesserung der Arterienheilung resultierte. Insbesondere waren die erfindungsgemäßen Extrakte geeignet, eine Neointima-Bildung zu reduzieren oder sogar zu verhindern.

Die erfindungsgemäßen Trockenextrakte aus Teufelskrallewurzeln wurden derart hergestellt, dass man Speicherwurzeln von Harpagophytum procumbens mit Wasser oder einem Gemisch aus Ethanol und Wasser extrahiert, den erhaltenen Extrakt konzentriert, die unlösliche von der erhaltenen löslichen Fraktion abtrennt und die erhaltene lösliche Fraktion trocknet.

Der erfindungsgemäße Trockenextrakt aus Teufelskrallenwurzel enthält weiterhin 2 Gew.-% gefälltes Siliciumdioxid als Fließregulierungsmittel. Der Trockenextrakt ist in Wasser trüb löslich und enthält ein Drogen-Extraktverhältnis von 2 bis 5 : 1, insbesondere von 1,5 bis 2 : 1. Der erfindungsgemäße Trockenextrakt enthält einen Harpagosid-Gehalt, gemessen mittels HPLC von 2 bis 3,2 Gew.-%.

Die erfindungsgemäßen Extrakte können unter Zusatz üblicher pharmazeutischer Hilfsstoffe in bekannter Weise zu oral applizierbaren festen und flüssigen Arzneimittelzubereitungen mit anti-arteriosklerotischer Wirkung verabreicht werden. In den erfindungsgemäßen Arzneimittelzubereitungen sind Wirkstoffgehalte von 70-3500 mg, insbesondere von 400-800 mg, Trockenextrakt aus Harpagophytum procumbens enthalten. Gegebenenfalls kann zusätzlich ein Trockenextrakt aus Weidenrinde in Konzentrationen von 60-1200 mg, insbesondere von 120-600 mg enthalten sein.

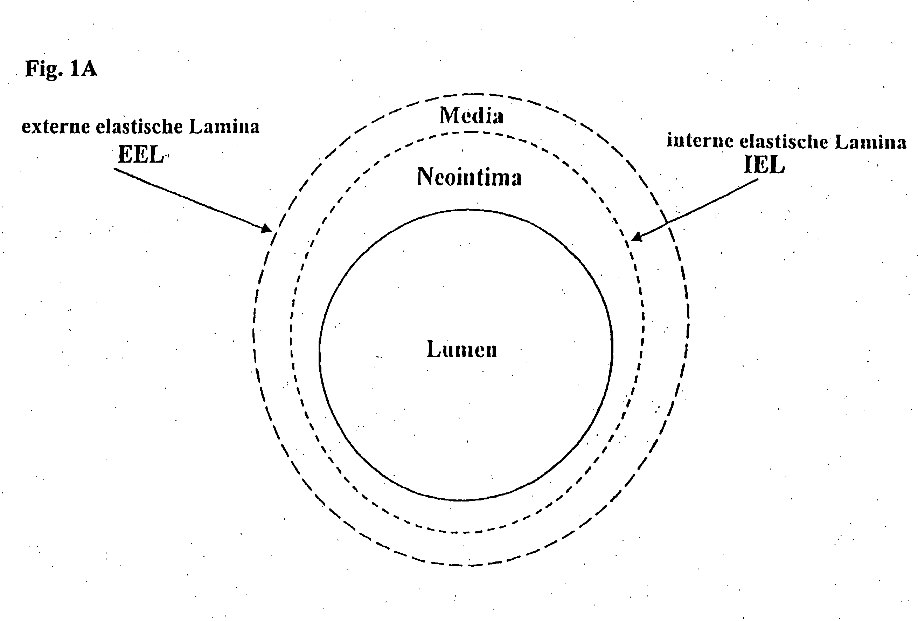

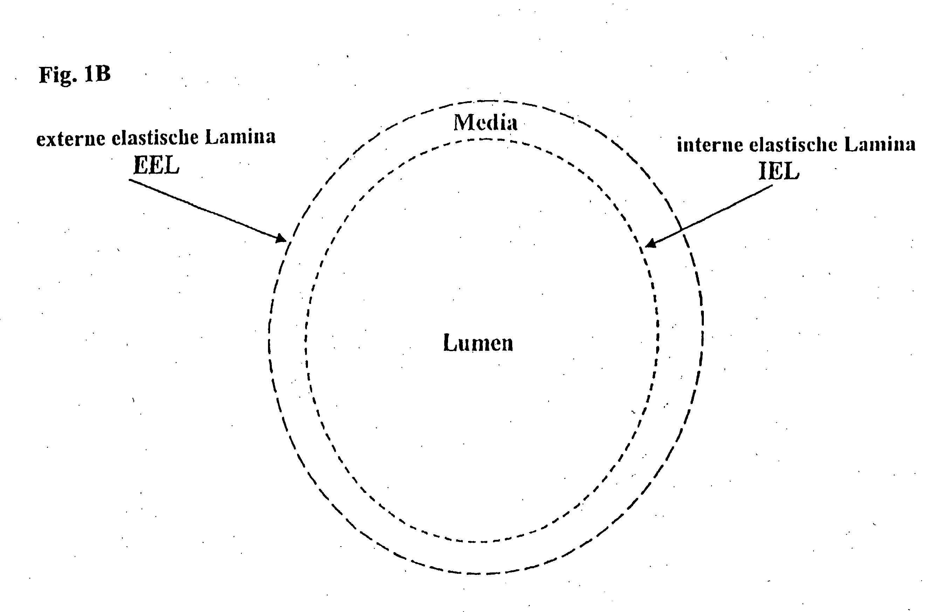

Die Erfindung wird nun anhand der Beispiele 1 bis 4 und der Figuren 1 bis 3 näher erläutert, ohne diese jedoch einzuschränken.

Figuren 1A und 1B zeigen eine schematische Darstellung der Anatomie der A. femoralis einer Maus zwei Wochen nach der arteriellen Verletzung (Figur 1A) und einer nicht verletzten Arterie (Figur 1B). Es wird das Lumen gezeigt, welches den arteriellen Blutfluss erlaubt; die Media als hauptsächlicher Bestandteil der normalen arteriellen Wand, die zwei deutlichen, die Media innerhalb und außerhalb abgrenzenden Laminae und die neu gebildete Neointima in Figur 1A.

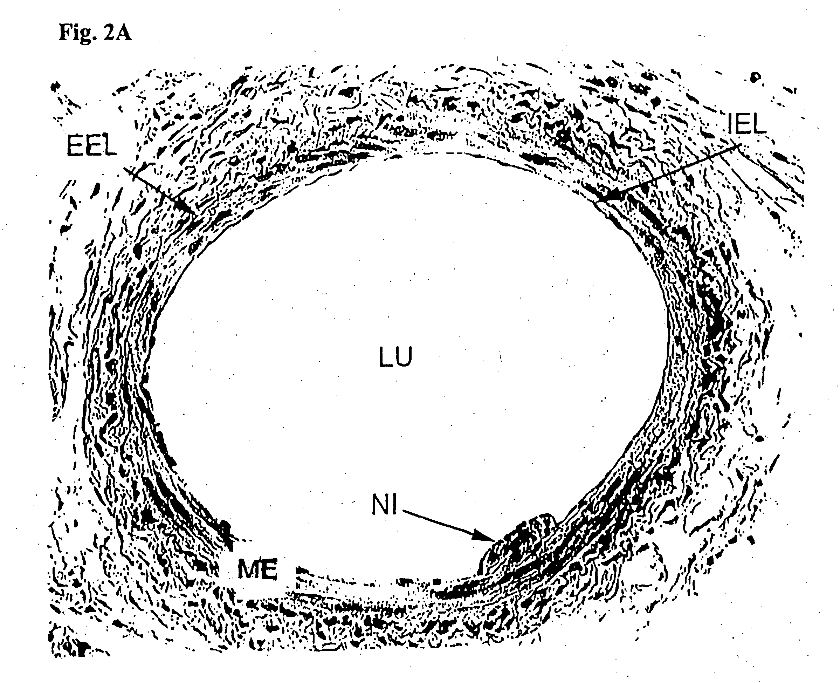

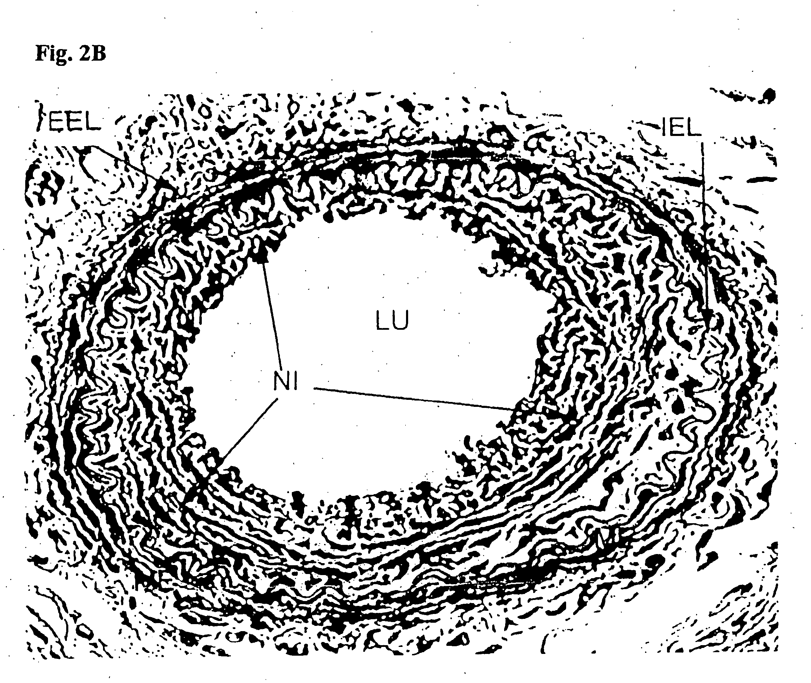

Fig. 2A und 2B zeigen jeweils mikroskopische Bilder von mit Hämatoxylin-Eosin gefärbten Schnitten der Oberschenkel-arterien von Mäusen zwei Wochen nach der arteriellen Verletzung aufgenommen mit 400facher Vergrößerung. Figur 2A zeigt ein größtenteils unbehindertes Lumen (LU) und nur minimale Neointima(NI)-Bildung in Arterie eines mit Harpagophytum procumbens behandelten Tieres. Die durch interne und externe Lamina (IEL und EEL) abgegrenzte Media (ME) zeigt normale Größe. Im Gegensatz kann zu dem gleichen Zeitpunkt in Figur 2B bei einer verletzten Oberschenkelarterie eines nicht behandelten Tieres eine bedeutende Lumenverengung und eine übermäßige Neointima-Bildung gesehen werden.

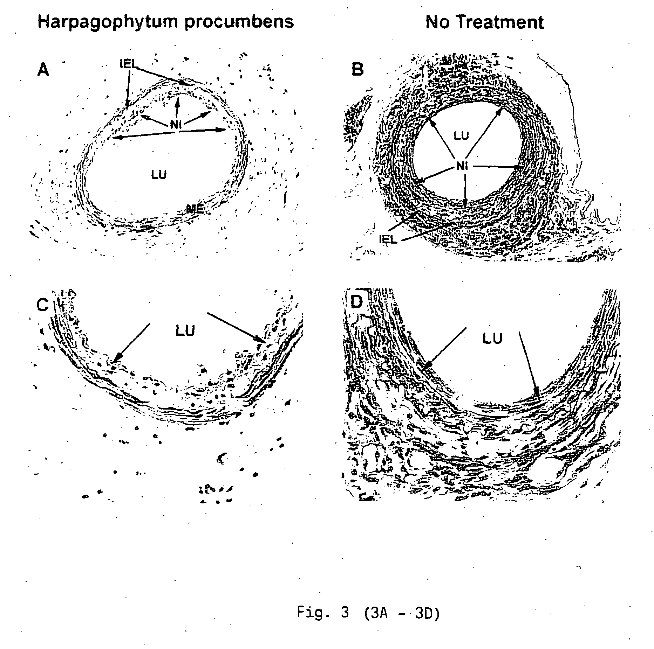

Fig. 3A bis 3D zeigen mikroskopische Bilder von Hematoxylen-Eosin angefärbten Abschnitten von femoralen Mäusearterien, zwei Wochen nachdem die arterielle Verletzung stattgefunden hat, mit einer Vergrößerung von x 400.

Fig. 3A zeigt ein sehr großes, unverschlossenes Lumen (LU) und eine nur geringe Neointima(NI)-Bildung in der Arterie eines Tieres, das mit Harpagophytum procumbens vier Wochen lang behandelt wurde. Die Media (ME), die zwischen der internen und externen elastischen Lumina (IEL und EEL) sich befindet, zeigt eine normale Größe. Im Gegensatz dazu wird in Fig. 3B ein signifikanter Lumenverschluss beobachtet, sowie eine exzessive Neointima-Bildung in der verletzten femoralen Arterie von nicht behandelten Tieren (Fig. 3B). Eine sehr hohe Auflösung zeigt in den Fig. 3C und 3D von angefärbten Schnitten, dass die Zyto-Architektur in der Neointima (NI) signifikant durch die Harpagophytum procumbens Behandlung verändert wird.

### Beispiel 1

989,9 kg getrocknete sekundäre Speicherwurzeln von Harpagophytum procumbens wurden durch Mahlen zerkleinert und mit 40 % Ethanol/Wasser während 0,5 bis 4 Stunden bei 20 bis 70°C extrahiert. Anschließend wurde konzentriert und der erhaltene Primärextrakt bei Raumtemperatur mit Ethanol 96,5 % ausgerührt. Die lösliche Fraktion wurde von der unlöslichen Fraktion abgetrennt und getrocknet. Es resultierte ein harpagosidreicher Trockenextrakt mit einem Harpagosid-Gehalt von 2,3 - 3,3 % (mittels HPLC bestimmt).

### Beispiel 2

277,6 kg geschnittene, getrocknete Weidenringe junger Zweige wurden mit 70 Gew.-% Ethanol unter Rühren auf 50°C erwärmt. Danach wurde der Rückstand abfiltriert und nochmals mit Ethanol 50 Gew.-% bei 50°C extrahiert. Die Extraktlösungen wurden in einem Zentrifugalverdampfer konzentriert und anschließend in Vakuum getrocknet. Es resultierte ein salicinreicher Trokkenextrakt mit einem Salicin-Gehalt von 12 - 20% (mittels HPLC bestimmt). Der Trockenextrakt fiel als braunes Pulver an und war in Wasser löslich.

### Beispiel 3

### Filmtabletten mit erfindungsgemäßem Extrakt aus Teufelskrallenwurzel

Filmtabletten mit 400 mg bis 2000 mg Drogenäquivalent (gesamte mögliche Menge an Droge), weisen folgende Zusammensetzung auf:

| | | |
|---|---|---|
| Trockenextrakt aus Teufelskrallenwurzel von Beispiel 1 | 2000 mg | 400 mg |
| Hochdisperses Siliciumdioxid | 5 mg | 10 mg |
| Polyvinylpyrrolidon (Molekulargewicht 25.000) | 3 mg | 6 mg |
| Quervemetztes Polyvinylpyrrolidon | 5 mg | 10 mg |
| Magnesiumstearat | 2 mg | 4 mg |
| Cellulose, mikrokristallin | 35 mg | 70 mg |

Die Bestandteile wurden gemischt und zu Tabletten verpresst. Die Tabletten wurden mit einem Filmüberzug auf Basis von Methylhydroxypropylcellulose überzogen.

### Beispiel 4

### Filmtabletten mit Trockenextrakt aus Teufelskrallenwurzel und Trockenextrakt aus Weidenrinde

Filmtabletten mit 400 mg bis 2000 mg Drogenäquivalent aus Teufelskrallenwurzel und 600 mg bis 3000 mg Drogenäquivalent aus Weidenrinde weisen folgende Zusammensetzung auf:

| | | |
|---|---|---|
| Trockenextrakt aus Teufelskrallenwurzel von Beispiel 1 | 200 mg | 400 mg |
| Trockenextrakt aus Weidenrinde von Beispiel 2 | 120 mg | 240 mg |
| Hochdisperses Siliciumdioxid | 7,5 mg | 15 mg |
| Polyvinylpyrrolidon (Molekulargewicht 25.000) | 5 mg | 9 mg |
| Quervernetztes Polyvinylpyrrolidon | 7,5 mg | 15 mg |
| Magnesiumstearat | 4 mg | 6 mg |
| Cellulose, mikrokristallin | 50 mg | 105 mg |

Die Bestandteile wurden vermischt und zu Tabletten verpresst. Die Tabletten wurden mit einem Filmüberzug auf Basis von Methylhydroxypropylcellulose überzogen.

### Pharmakologische Wirksamkeit der erfindungsgemäßen Extrakte

Die pharmakologischen Effekte der Auswirkungen von Harpagophytum procumbens (Teufelskralle) wurden an einem Mausmodell bei arterieller Verletzung untersucht. Es konnte nachgewiesen werden, dass ansteigende Harpagophytum procumbens-Extrakt-Gaben die Neointima-Bildung, welche auf die endothele Denudation der Arteria femoralis (A. femoralis) folgt, vorteilhaft regulieren.

Erfindungsgemäß wurde festgestellt, dass steigende Dosen von Harpagophytum procumbens (Teufelskralle)-Extrakt die Neointima-Bildung, welche auf die endotheliale, denudierende Verletzung der Maus folgt, vorteilhaft regulieren können.

Um die Auswirkungen der erfindungsgemäßen Zubereitung mit Harpagophytum procumbens (Teufelskralle-Extrakt) auf die Neointima-Bildung zu bewerten, wurden Neointima-Bereiche, das Neointima/Media-Verhältnis [Verhältnis von Neointima (NI)-Bereich zu Media (ME)-Bereich], Stenosegrad [Verhältnis von Neointima-Bereich zu einem Bereich, der durch einen internen, elastischen Lamina (IEL)-Bereich begrenzt ist] und einen umgeformten Index [Verhältnis von Neointima-Bereich zu einem Bereich, der durch einen externen, elastischen Lamina-Bereich begrenzt ist] in geschädigten bzw. verletzten A. femoralis von Tieren mit (n = 11, n = 14) und ohne (n = 10; n = 29) Behandlung mit dem erfindungsgemäßen Teufelskralle-Trockenextrakt verglichen (vgl. Fig. 1A, Fig. 1B).

### Methoden

### Mäuse

3 bis 4 Monate alte, männliche C57BL/6-Mäuse wurden von Taconic Farms erworben und in dem Center for Laboratory Animal Sciences im The Mount Sinai Medical Center, New York, NY untergebracht. Die Mäuse erhielten Standard-Nagetierfutter (Mäusenahrung # 5015, PMI Nutrition International) und Leitungswasser antirheumatisch. Vorgehensweise und Tierpflege sind vom "Institutional Animal Care and Use Committee" anerkannt und waren in Übereinstimmung mit dem "Guide for the Care and Use of Laboratory Animals" (National Research Council. Washington, D.c: National Academy Press 1996).

### Harpagophytum procumbens-Verabreichung und endotheliale Denudations-Verletzung von Maus-A. femoralis

Zwei Test-Gruppen von C57/BL6-Mäusen wurden an der A. femoralis verletzt. Eine Gruppe erhielt Harpagophytum procumbens-Extrakt (n = 25), während die andere Gruppe (n = 38) keine Behandlung erfuhr. In Phosphat-gepufferter Salzlösung (PBS) gelöster Harpagophytum procumbens-Extrakt wurde eine Woche vor der arteriellen Verletzung verabreicht und dann für einen Zeitraum von zwei Wochen (n = 11) oder von vier Wochen (n = 14) mit einer Dosis von 50 mg/kg *per die* durch *i*.*p*.-Injektionen fortgesetzt. Zusätzlich wurde den Tieren in der ersten Behandlungsgruppe 1 mg/ml Harpagophytum procumbens-Extrakt (gemäß Beispiel 1) enthaltendes Trinkwasser mit dem Beginn des Behandlungszeitraums (Verletzung der A. femoralis) zugeführt. Auf Basis eines täglich Trinkwasserverbrauchs von ungefähr 6ml bei unter diesen Bedingungen gehalten Mäusen und einem Durchschnittsgewicht von 20g pro Maus ergibt dies eine Harpagophytum procumbens-Dosis (oral) von etwa 300mg/kg *per die.*

Zur Vorbereitung der arteriellen Verletzung wurde den Versuchstieren eine Vollnarkose durch intraperitoneale Natriumpentobarbital-Injektionen (Nembutalâ, Abbott Laboratories) mit einer Dosis von 40 mg/kg Körpergewicht verabreicht. Die Verletzung an der A. femoralis wurde durch dreimaliges Einführen eines 0,25mm angioplastischen Führungsdrahts bzw. Herzkatheters (Advanced Cardiovascular Systems) und Entfernen des Endothels mittels einer Raspel erzeugt. Ein Operationsmikroskop (Carl Zeiss) wurde für die Operation verwendet. Sowohl das Protokoll als auch der Grad der an der Gefäßwand angebrachten Verletzung sind standardisiert.

### Histologie

Die Tiere werden mit 4 % Paraformaldhyd in PBS bei 100mm Hg für 10 Minuten Perfusionsfixiert und ihre unteren Gliedmaßen en bloc entfernt. Proben wurden über Nacht in 4 % Paraformaldehyd in PBS fixiert und in 10 % Ameisensäure entkalkt. Zwei 2mm dicke Querschnitte wurden von jeder Gliedmaße auf der Höhe der Verletzung in der gemeinen Oberschenkelarterie geschnitten und zum Einlegen in Paraffin verarbeitet. Aufeinanderfolgende Schnitte (4mm dick) werden erhalten und mit Hämatoxylin-Eosin eingefärbt.

### Morphometrie

Um die Auswirkungen des erfindungsgemäßen Arzneimittels mit Harpagophytum procumbens (Teufelskralle-Extrakt) auf die Neointima-Bildung zu beurteilen, wurden Neointima-Bereich, Neointima/Media-Verhältnis [Verhältnis von Neointima (NI)-Bereich zu Media (ME)-Bereich], Stenosegrad [Verhältnis von Neointima-Bereich zu einem Bereich, der durch einen internen, elastischen Lamina (IEL)-Bereich begrenzt ist] und einen Umformumgsindex [Verhältnis von Neointima-Bereich zu einem Bereich, der durch einen externen, elastischen Lamina-Bereich begrenzt ist] in verletzten Oberschenkelarterien von mit Harpagophytum procumbens (n = 25) behandelten und unbehandelten (n = 38) Tieren verglichen.

Die Auswertung wurde von unbeeinflussten Personen durchgeführt. Weiterhin wurde ein computergestütztes Planimetrie-System wurde verwendet (Software: Image Pro Plus 3.0.1). Zur Bewertung des Neointima-Bereichs, des IEL-Bereichs, des Media-Bereichs und des EEL-Bereichs wurden mit Hämatoxylin-Eosin eingefärbte Schnitte untersucht. Das Neointima/ Media (NI/ME)-Verhältnis, der Verengungsgrad [Verhältnis von Neointima-Bereich zu einem Bereich, der durch einen internen, elastischen Lamina (IEL)-Bereich begrenzt ist] und ein Umformumgsindex [Verhältnis von Neointima-Bereich zu einem Bereich, der durch einen externen, elastischen Lamina-Bereich begrenzt ist] wurden wie vorhergehend beschrieben berechnet und die einzelnen Bereiche wie auch die anatomischen Orientierungspunkte werden in Figur 1A (schematische Zeichnung einer verletzten Arterie zwei Wochen nach der endothelen Denudation) und Figur 1B (schematische Zeichnung einer nicht verletzten Arterie ohne Neointima zum Vergleich) aufgezeigt.

### Statische Untersuchung

Zur Datenuntersuchung wurde die SPSS/PC+-Software verwendet. Die Daten werden als Mittel ± SEM gegeben. Nach dem Untersuchen auf eine Normalverteilung und auf Gleichheit der Abweichungen mit dem F-Test von Levene, wurde der unabhängige Proben t- Test verwendet, um Neointima-Bereich, IEL-Bereich, Media-Bereich, EEL-Bereich, Neointima zu Media-(NI/ME)-Verhältnis, Verengungsgrad [Verhältnis von Neointima-Bereich zu einem Bereich, der durch einen internen, elastischen Lamina (IEL)-Bereich begrenzt ist] und den Umformungsindex [Verhältnis von Neointima-Bereich zu einem Bereich, der durch einen externen, elastischen Lamina-Bereich begrenzt ist] zu vergleichen. Die Wahrscheinlichkeitswerte wurden auf zwei gültige Stellen angegeben. Wahrscheinlichkeitswerte (p) von < 0,05 wurden als bemerkenswert betrachtet.

### Ergebnisse

### Grad der Neointima-Bildung

Der Neointima-Bereich wurde zwei Wochen nach der arteriellen Verletzung um mehr als 50 % mit der Harpagophytum procumbens (Teufelskralle-Extrakt)-Behandlung in Vergleich zu den nicht behandelten Tieren vermindert und bei einigen mit Harpagophytum procumbens (Teufelskralle) behandelten Tieren wurde die Bildung von Neointima fast vollständig verhindert (8,07 ± 1,76 x 10⁻³ mm² gegenüber 9,87 ± 2,93 x 10⁻³ mm²; p = 0,6; Figur 2A).

Jedoch ist festzustellen, dass der Neointima-Bereich vier Wochen nach der arteriellen Verletzung signifikant erniedrigt wurde mit der Harpagophytum procumbens (Teufelkralle-Extrakt-) Behandlung im Vergleich zu den nicht behandelten Tieren und die Neointima-Bildung bei einigen Tieren, die mit Harpagophytum procumbens behandelt worden waren, bzw. einer Kombination aus Harpagophytem procumbens (Weidenrinde-Extrakt), konnte gänzlich verhindert werden (4,87 ± 1,17 10⁻³ mm² gebenüber 7,71 ± 0,93 x 10⁻³ mm²; p < 0,005; Figuren 2A und 3).

Folglich wurde das Neointima-Media Verhältnis (NI/ME), welches die Neointima-Größe reflektiert und im Bezug die Gesamtgröße der Arterie korrigiert, mehr als 50 % von 1,16 ± 0,46 bei nicht behandelten Tieren auf 0,44 ± 0,35 bei mit Harpagophytum procumbens behandelten Tieren reduziert (p < 0,01) (siehe Fig. 2B).

### Arterielle Beengungen und arterielle Umbildungen

Der durch das Verhältnis zwischen Neointima-Bereich und IEL-Bereich bewertete Verengungsgrad wurde, wie in Fig. 2C gezeigt, durch die fortwährende Verabreichung von Harpagophytum procumbens (Teufelskralle-Extrakt) deutlich reduziert, verglichen mit der nicht behandelten Gruppe (15 ± 3 % gegenüber 23 ± 3 %, p < 0,005). Der durch das Verhältnis zwischen Neointima-Bereich und IEL-Bereich definierte Umformungsindex wurde folglich verringert (9 ± 2 % gegenüber 16 ± 4 %, p = 0,089).

### Schlussfolgerungen

Die fortwährende Verabreichung von Harpagophytum procumbens (Teufelskralle-Extrakt) mit einer Dosierung von 50 mg/kg *per die,* gegeben durch *i*.*p*.-Injektionen und mit einer Dosierung von 300 mg/kg *per die* gegeben *per os* erlaubt es, den Prozess der Neointima-Bildung nach Verletzung der A. femoralis bei Mäusen vorteilhaft zu regulieren. Alle zur Beurteilung der arteriellen Antwort auf die Verletzung verwendeten Parameter zeigten vier Wochen nach der endothelen, denudierenden Verletzung auf eine große Verbesserung der Arterienheilung durch Harpagophytum procumbens in der gegebenen Dosis. Unterschiede von mehr oder nahe bei 50 % bei diesen Schlüsselgrößen zwischen der behandelten und der nicht behandelten Gruppe mit p-Werten dicht oder unter 0,05 legen gewöhnlich eine biologische Wirksamkeit eines Medikaments nahe. Weiterhin kann eine interessante Veränderung der Cyto-Architektur der Neointima nach vierwöchiger Verabreichung von Harpagophytum procumbens-Extrakten festgestellt werden, wie dies in den Figuren 3C und 3D festzustellen ist.

## Patentansprüche

1. Verwendung von Extrakten aus Harpagophytum procumbens (Teufelskralle), gegebenenfalls in Kombination mit Extrakten aus Salicis cortex (Weidenrinde), zur Herstellung eines Arzneimittels zur Behandlung von Arteriosklerose.

2. Verwendung gemäß Anspruch 1, **dadurch gekennzeichnet, dass** die Extrakte aus Harpagophytum procumbens erhältlich sind durch Extrahieren der Droge mittels Wasser oder eines Gemisches aus Ethanol und Wasser, Konzentrieren des erhaltenen Extraktes, Abtrennen der unlöslichen von der erhaltenen löslichen Fraktion und Trocknen der erhaltenen löslichen Fraktion.

3. Verwendung gemäß Anspruch 1 oder 2, **dadurch gekennzeichnet, dass** die Extrakte einen Harpagosid-Gehalt von 2,0 - 3,2 Gew.-% aufweisen.

4. Verwendung gemäß einem der vorhergehenden Ansprüche 1 bis 3, **dadurch gekennzeichnet, dass** sie einen Trockenextraktgehalt aus Harpagophytum procumbens von 70 - 3500 mg, insbesondere von 400 - 800 mg, aufweist.

5. Verwendung gemäß einem der Ansprüche 1 bis 4, **dadurch gekennzeichnet, dass** die Extrakte aus Salicis cortex durch Extrahieren der Droge mittels Wasser oder eines Gemisches aus Ethanol und Wasser, Konzentrieren der erhaltenen Extrakte, Abtrennen der unlöslichen von der rhaltenen löslichen Fraktion und Trocknen der erhaltenen löslichen Fraktion erhältlich sind.

6. Verwendung gemäß einem der Ansprüche 1 bis 5, **dadurch gekennzeichnet, dass** die Extrakte einen Salicin-Gehalt von 12 - 15 Gew.-% aufweisen.

7. Verwendung gemäß einem der Ansprüche 1 bis 6, **dadurch gekennzeichnet, dass** das Arzneimittel 60 - 1200 mg, insbesondere 600 - 1200 mg, Trockenextrakt aus Salicis cortex enthält.

8. Verwendung gemäß einem der vorhergehenden Ansprüche 1 bis 7, **dadurch gekennzeichnet, dass** das Arzneimittel in Form einer oral applizierbaren festen oder flüssigen Arzneimittelzubereitung vorliegt.

## Claims

1. Use of extracts of Harpagophytum procumbens (devil's claw), possibly in combination with extracts of Salicis cortex (willow bark), to produce a pharmaceutical medicament for the treatment of arteriosclerosis.

2. Use according to claim 1, **characterised in that** the extracts of Harpagophytum procumbens are available by extracting the drug by means of water or a mixture of ethanol and water, concentrating the obtained extract, separating the insoluble fraction from the obtained soluble fraction and drying the obtained soluble fraction.

3. Use according to claim 1 or 2, **characterised in that** the extracts have a harpagoside content of 2.0 - 3.2 % by wt.

4. Use according to one of the preceding claims 1 to 3, **characterised in that** it has a dried extract content of Harpagophytum procumbens of 70 - 3500 mg, more especially 400 - 800 mg.

5. Use according to one of claims 1 to 4, **characterised in that** the extracts of Salicis cortex are available by extracting the drug by means of water or a mixture of ethanol and water, concentrating the obtained extracts, separating the insoluble fraction from the obtained soluble fraction and drying the obtained soluble fraction.

6. Use according to one of claims 1 to 5, **characterised in that** the extracts have a salicine content of 12 - 15 % by wt.

7. Use according to one of claims 1 to 6, **characterised in that** the pharmaceutical medicament contains 60 - 1200 mg, more especially 600 - 1200 mg, dried extract of Salicis cortex.

8. Use according to one of the preceding claims 1 to 7, **characterised in that** the pharmaceutical medicament is in the form of an orally applicable solid or liquid pharmaceutical medicament preparation.

## Revendications

1. Utilisation d'extraits d'*Harpagophytum procumbens* (Nom commun : Griffe du Diable) éventuellement en combinaison avec des extraits de *Salicis cortex* (écorce de saule), pour la préparation d'un médicament destiné au traitement de l'artériosclérose.

2. Utilisation selon la revendication 1, **caractérisée en ce que** les extraits d'*Harpagophytum procumbens* peuvent être obtenus par extraction de la drogue au moyen d'eau ou d'un mélange d'éthanol et d'eau, concentration de l'extrait obtenu, élimination de la fraction insoluble par séparation de la fraction soluble obtenue et séchage de la fraction soluble obtenue.

3. Utilisation selon l'une des revendications 1 ou 2, **caractérisée en ce que** les extraits présentent une teneur en harpagoside de 2,0 à 3,2 % en poids.

4. Utilisation selon l'une quelconque des revendications 1 à 3, **caractérisée en ce qu'**elle présente une teneur en extrait sec d'*Harpagophytum procumbens* de 70-3500 mg, et plus particulièrement de 400-800 mg.

5. Utilisation selon l'une quelconque des revendications 1 à 4, **caractérisée en ce que** les extraits de *Salicis cortex* peuvent être obtenus par extraction de la drogue au moyen d'eau ou d'un mélange d'éthanol et d'eau, concentration des extraits obtenus, élimination de la fraction insoluble par séparation de la fraction soluble obtenue et séchage de la fraction soluble obtenue.

6. Utilisation selon l'une quelconque des revendications 1 à 5, **caractérisée en ce que** les extraits présentent une teneur en salicine de 12-15 % en poids.

7. Utilisation selon l'une quelconque des revendications 1 à 6, **caractérisée en ce que** le médicament contient 60-1200 mg, et plus particulièrement 600-1200 mg d'extrait sec de *Salicis cortex.*

8. Utilisation selon l'une quelconque des revendications 1 à 7, **caractérisée en ce que** le médicament se présente sous forme d'une préparation médicamenteuse solide ou liquide administrable par voie orale.
